(19) Europäisches Patentamt
European Patent Office
Office européen des brevets

(11) Veröffentlichungsnummer: **0 014 898**
**A1**

(12) **EUROPÄISCHE PATENTANMELDUNG**

(21) Anmeldenummer: **80100597.6**

(22) Anmeldetag: **06.02.80**

(51) Int. Cl.³: **C 12 Q 1/30**
C 12 Q 1/26, C 12 Q 1/54

(30) Priorität: **27.02.79 DE 2907628**

(43) Veröffentlichungstag der Anmeldung:
**03.09.80 Patentblatt 80/18**

(84) Benannte Vertragsstaaten:
**AT BE CH FR GB IT LU NL SE**

(71) Anmelder: **C.A. Greiner & Söhne GmbH & Co. KG**
**Galgenbergstrasse 9c**
**D-7440 Nürtingen(DE)**

(72) Erfinder: **Häbich, Hans-Joachim**
**Tannenstrasse 10**
**D-7031 Hildrizhausen(DE)**

(74) Vertreter: **Dreiss, Uwe, Dr. jur. Dipl.-Ing. M.Sc et al,**
**Patentanwälte Dreiss & Fuhlendorf Schickstrasse 2**
**D-7000 Stuttgart 1(DE)**

(54) **Proberöhrchen für die Untersuchung von Proben im klinischen Bereich, insbesondere von Urinproben.**

(57) Proberöhrchen (1) für die Untersuchung von Proben im klinischen Bereich, insbesondere von Urinproben, wobei die durch die Probe festzustellende Substanz mit einem ersten Reagenz unter Abgabe von $H_2O_2$ reagiert und das dadurch gebildete $H_2O_2$ durch Oxidation eines Farbstoffbildners zur Anzeige verwendet wird. Störungseinflüsse durch andere Substanzen, die ebenfalls mit $H_2O_2$ reagieren, enthält das Proberöhrchen eine

a) Katalase,
b) eine unter dem Einfluß von Katalase Sauerstoff abgebende Substanz, sowie eine
c) Oxidase.

Katalase und Oxidase sind in eine Schicht (2) an der Innenwand des Röhrchen eingebettet. Bei Eingeben der Probe gibt nun die Substanz (b), z.B. ein Peroxid, unter dem Einfluß der Katalase Sauerstoff ab, der die Störungssubstanz (z.B. Ascorbinsäure) oxidiert und damit den Einfluß auf die Farbindikation ausschaltet.

EP 0 014 898 A1

./...

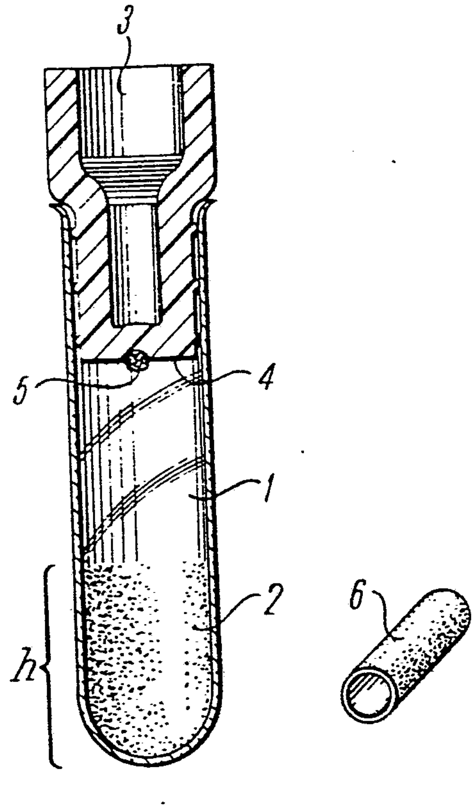
3
5
4
1
2
6
h

## Proberöhrchen für die Untersuchung von Proben im klinischen Bereich, insbesondere von Urinproben

Die Erfindung betrifft ein Proberöhrchen für Untersuchungen von Proben im klinischen Bereich, insbesondere von Urinproben, unter Verwendung eines ersten Reagenz, das mit einer in der Probe vorhandenen festzustellenden Substanz unter Abgabe von $H_2O_2$ reagiert, und eines zweiten Reagenz, das mit Hilfe des derart gebildeten $H_2O_2$ die Oxidation eines Farbstoffbildners zum Farbstoff bewirkt, der ein Indikator für die festzustellende Substanz ist.

Ein Beispiel für eine derartige Untersuchung ist die Glucose-Anzeige bei Teststreifen. Das erste Reagenz ist dabei Glucoseoxidase, die in Anwesenheit von Sauerstoff (in der Luft) die Glucose zu δ-Gluconolacton oxidiert. Dabei wird Wasserstoffperoxid ($H_2O_2$) frei. Es läuft also folgende Reaktion ab:

Reaktion (1):

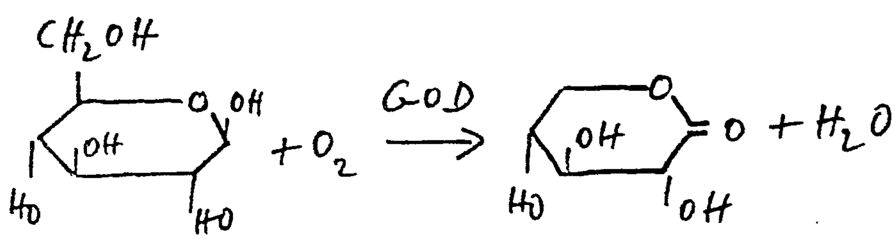


D-Glucose δ-Gluconolactan

Das bei dieser Reaktion (1) gebildete Wasserstoffperoxid oxidiert nun die farblose bei Oxidation farbstoffbildende Substanz, also z.B. ein farbloses Chromogen ($FH_2$) wie o-Tolidin, zum Farbstoff (F), z.B. "o-Tolidin-blau". Diese Reaktion läuft in Anwesenheit von Peroxidase (POD) wie folgt ab:

Reaktion (2):

$$H_2O_2 + FH_2 \xrightarrow{POD} 2H_2O + F$$

reduzierter Farbstoff, farblos — Farbstoff Färbung sichtbar

Es ist nun bekannt (s. Kohl, Störungen der Glucose-Anzeige durch Ascorbinsäure bei Teststreifen, Laboratoriumsblätter 27 (1977), S.103-108), daß die Anwesenheit von Ascorbinsäure in der Urinprobe zu falsch-negativen Reaktionen führt, da das bei der Oxidation von Glucose zu $\delta$-Gluconolacton frei werdende Wasserstoffperoxid, dessen Menge ja nach Reaktion (2) das Ausmaß der Färbung des Teststreifens bestimmt, mit der Ascorbinsäure reagiert und diese zu Dehydro-Ascorbinsäure oxidiert. Dies ist eine farblose Substanz. Die Reaktion läuft wie folgt ab:

Reaktion (3):

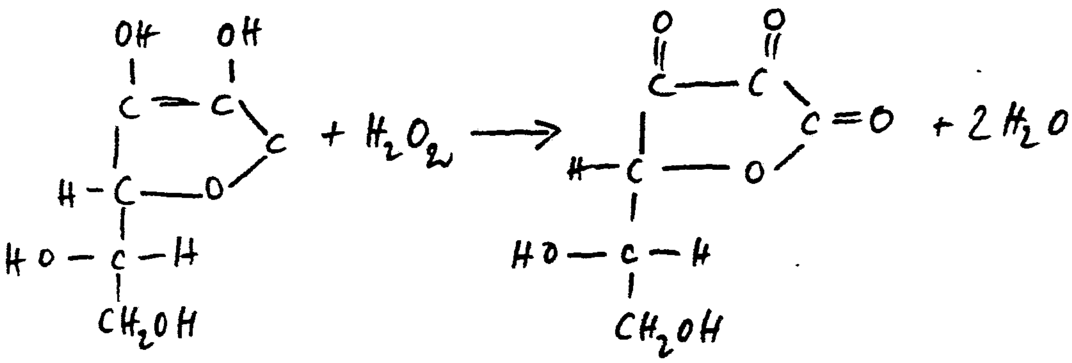

L-Ascorbinsäure — L-Dehydro-Ascorbinsäure

Das heißt in anderen Worten: Das bei der Reaktion (1) entstehende Wasserstoffperoxid ist bereits durch die Reaktion (3) mit der Ascorbinsäure verbraucht und führt daher nicht mehr zur Farbstoffbildung und daher nicht mehr zur Anzeige der Anwesenheit von Glucose in der Urinprobe, so daß die Anwesenheit von Glucose nicht mehr bzw. nicht in einem den tatsächlichen Verhältnissen entsprechenden Ausmaß erfolgt.

Um bei der Feststellung von Glucose mit Teststreifen diesen Fehler auszuschalten, ist nun vorgeschlagen worden, in Teststreifen ferner einen Ascorbinsäure-Testbezirk vorzusehen (Kohl, a.a.o.,S.105), so daß die Auswertung von Proben, die Ascorbinsäure enthalten, verhindert werden kann. Diese Methode führt jedoch nur zur Erkennung von solchen Urinproben, die für die Durchführung der Schnelltests mit Teststreifen ungeeignet sind.

Aufgabe der Erfindung ist es, ein Proberöhrchen zu schaffen, in dem derartige Untersuchungen durchgeführt werden können, ohne daß in der Probe ebenfalls enthaltene Störsubstanzen, die mit dem $H_2O_2$ reagieren, die Untersuchung verfälschen. Von dieser Aufgabenstellung wird z.B. die Schaffung eines Urinproberöhrchens umfaßt, in dem der herkömmliche Glucose-Test mit Hilfe von Teststreifen auch dann fehlerfrei ablaufen soll, wenn die Urinprobe Ascorbinsäure enthält.

Diese Aufgabe wird bei einem Proberöhrchen der eingangs gegenannten Art erfindungsgemäß dadurch gelöst, daß das Röhrchen

a) Katalase,
b) eine unter dem Einfluß von Katalase Sauerstoff abgebende Substanz, sowie
c) eine Oxidase, die in Gegenwart des Sauerstoffs die Störungssubstanzen oxidiert,

enthält, wobei die Katalase und die Oxidase eine Schicht bilden bzw. in einer Schicht eingebettet sind, die einen

Teil der Innenwandung des Röhrchens bedeckt, und daß ferner die Sauerstoff abgebende Substanz als Trockensubstanz in dem Röhrchen enthalten ist.

Eine Ausführungsart der Erfindung betrifft ein Urinprobenröhrchen. Bei diesem ist die unter dem Einfluß von Katalase Sauerstoff abgebende Substanz ein Peroxid, z.B. Carbamid-Peroxid; ferner ist die Oxidase die Ascorbinsäure-Oxidase. Sobald also die Urinprobe in das Röhrchen eingegeben wird, bildet die Katalase aus dem Peroxid Sauerstoff und Wasser. Dies erfolgt nach folgender Reaktion:

Reaktion (4):

$$2H_2O_2 \xrightarrow{\text{Katalase}} 2H_2O + O_2$$

Diese Reaktion läuft erst im Zeitpunkt des Eingebens der Urinprobe ab, da die Katalase, die mit der Ascorbinsäure-Oxidase die Schicht bildet, und das Peroxid separat trocken in dem Röhrchen vorhanden sind. Die Reaktion (4) läuft also erst ab, sobald die Urinprobe eingefüllt wird.

Der durch die Reaktion (4) gebildete Sauerstoff oxidiert nun in Gegenwart der Ascorbinsäure-Oxidase (ASOD) nach der folgenden Reaktion die Ascorbinsäure zur Dehydro-Ascorbinsäure:

Reaktion (5):

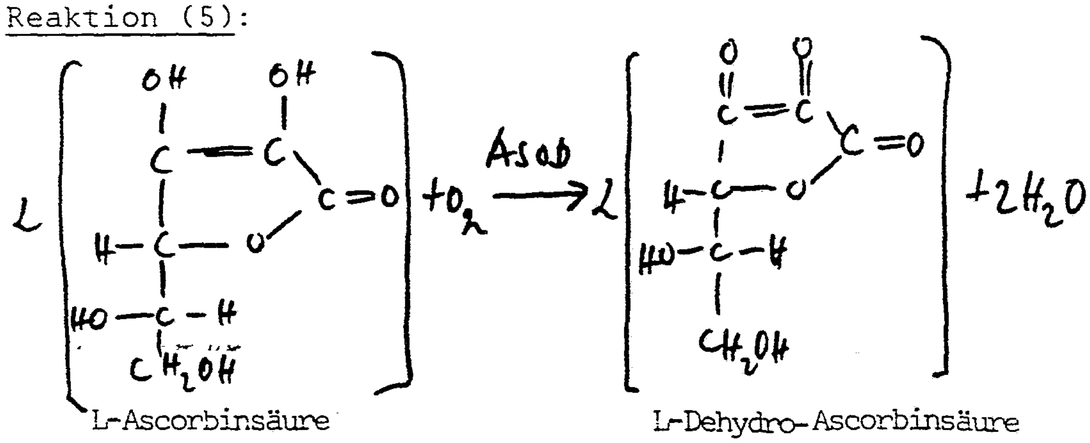


L-Ascorbinsäure L-Dehydro-Ascorbinsäure

Dadurch wird die in der Urinprobe vorhandene Ascorbinsäure so umgesetzt, daß das entstehende Oxidationsprodukt, nämlich die Dehydro-Ascorbinsäure nicht mehr mit dem Wasserstoffperoxid reagieren kann, das bei der Reaktion (1), die Bestandteil des Glucose-Tests ist, entsteht. Voraussetzung dafür ist freilich, daß die Katalase in dem Röhrchen in solcher Menge vorhanden ist, daß sie bei Ablauf der Reaktion (4) das gesamte Peroxid, das sich in dem Röhrchen befindet, umsetzt.

Ein Ausführungsbeispiel der Erfindung wird nunmehr anhand der beigefügten Zeichnung beschrieben, die ein Ausführungsbeispiel darstellt. Das Röhrchen 1 ist von unten her bis zur Höhe h auf seiner Innenwandung mit einer Schicht 2 versehen, die Katalase und Ascorbin-Oxidase enthält. Das Röhrchen 1 ist mit einem Stopfen 3 verschlossen, der auf seiner der Innenseite des Röhrchens 1 zugewandten Stirnfläche 4 die Peroxidase trägt. Diese ist dort in Form einer Anhäufung 5 aufgetrocknet oder mit irgendeiner löslichen Substanz, z.B. Gelatine verklebt. Beim Einbringen einer Urinprobe löst sich zunächst die Schicht 2. Dann kann das Röhrchen gekippt werden, so daß auch die Peroxidase in die Urinprobe gelangt und sich dort löst. Dann laufen die Reaktionen (4) und (5) ab. Danach kann man dann in der bekannten Weise einen Teststreifen einbringen, auf dem die Reaktionen (1) und (2) zur Feststellung von Glucose ablaufen können, ohne daß diese durch die Störreaktion (3) beeinflußt oder negativ verfälscht wird.

Die Aufbringung der Schicht 2 kann z.B. folgendermaßen erfolgen. Man nimmt 75 mMol einer Pufferlösung, z.B. Tris, und löst sie in 1 l Wasser. Mit Zitronensäure wird diese Lösung auf pH=5,6 gebracht. Dieser Wert hat sich als besonders günstig für die Reaktion der Ascorbinsäure-Oxidase erwiesen.

In dieser Pufferlösung wird gelöst:

(1) Katalase. Diese Substanz ist im Handel erhältlich. Man löst pro Liter Pufferlösung 1000 mg Katalase.

(2) Ferner löst man in 1 l Pufferlösung 500 mg Ascorbinsäure-Oxidase. Die Herstellung dieser Substanz ist bekannt (vgl. Hoppe-Seyler/Thierfelder, Handbuch der physiologisch- und pathologisch-chemischen Analyse, 10. Aufl., 1964, S.909 ff.; s. ferner die Nachweise bei Boyer, Lardy, Myrbäck (Herausgeber), The Enzymes, 2. Aufl., Bd.8, New York und London (1963), S.297 ff.).

20 Mikroliter dieser Lösung benötigt man etwa für 15 ml einerUrinprobe. Man stellt also mit Hilfe von etwa 20 Mikroliter dieser Lösung, die durch das dargestellte Puffer/Enzym-Gemisch gebildet wird, die Schicht 2 auf der Innenwand des Röhrchens 1, dessen Volumen 15 ml Urin aufnimmt, her. Das kann z.B. mit Hilfe einer Vorrichtung erfolgen, die maschinell von oben her in das Innere des Röhrchens eingeführt wird und im Prinzip wie eine Sprühpistole arbeitet, wobei die aufgesprühte Menge der Flüssigkeit in der angegebenen Quantität begrenzt werden kann. Auf die Innenwandung des Röhrchens werden diese 20 Mikroliter des Puffer/Enzym-Gemisches aufgesprüht und das Röhrchen anschließend durch Hitzeeinwirkung getrocknet. Der feine Sprühnebel trocknet dann in der dargestellten Weise an der Innenwandung des Röhrchens 1 an.

Man muß dann noch Peroxid einbringen. Derartiges Peroxid, z.B. Carbamid-Peroxid ($CO(NH_2)_2 \cdot H_2O_2$) ist im Handel erhältlich. Wie bereits erwähnt, muß die Menge derart sein, daß sie durch die Katalase vollständig umgesetzt wird. Wählt man Katalase und Ascorbin-Oxidase in den oben angegebenen Mengen, so ist etwa eine Menge von 3 mg Peroxid zu wählen. in der in der beiliegenden Figur gezeigten Form ist diese Menge in räumlich konzentrierter Form, d.h. in Form einer Anhäufung 5 auf der Unterseite des Stopfens 3 angebracht.

Grundsätzlich gibt es außer der gezeigten Ausführungsart noch verschiedene Möglichkeiten zur Einbringung des Peroxids:

1. Man kann das Peroxid als Pulver ganz einfach in das Röhrchen schütten. Es bleibt dann entweder in pulveriger Form am Boden des Röhrchens liegen oder verteilt sich etwa gleichmäßig entlang der Innenwand an dem Röhrchen, beispielsweise aufgrund elektrostatischer Haftung o.dgl.. Die Menge ist auch so gering, daß im Prinzip nicht zu befürchten ist, daß das Peroxid etwa beim Öffnen des Röhrchens herausfällt.

2. Man kann aber auch das Peroxid in Wasser lösen oder in Alkohol suspendieren und als Schicht auf der Innenwandung des Röhrchens 1 oder auf der Stirnfläche des Stopfens 3 aufbringen. Dabei wird diese Schicht an der Innenwandung des Röhrchens 1 vorzugsweise an einer Stelle aufgebracht, an der sich die Schicht 2 nicht befindet. Gelingt es jedoch, beide Schichten übereinander aufzubringen, ohne daß sie miteinander während des Vorgangs des Aufbringens reagieren, so ist auch das im Prinzip möglich.

3. Eine weitere Möglichkeit bestünde darin, das Peroxid in einer schnell löslichen Gelatinekapsel in das Röhrchen einzubringen oder auch auf einen Träger, also etwa ein zylindrisches Stück Röhrchen 6 aufzubringen (z.B. durch Aufsprühen und Trocknen, wie oben beschrieben), welches Röhrchen 6 dann in das Innere des Röhrchens 1 eingegeben wird. Das Röhrchen 1 in der dargestellten Form, jedoch anstelle der Anhäufung 5 mit dem Röhrchen 6 versehen, bildet also eine Alternative.

Bringt man also das Peroxid derart ein, daß man es zunächst löst und dann an irgendeiner Stelle aufträgt oder aufsprüht und anschließend trocknen läßt, so kann man die Lösung beispielsweise so herstellen, daß man etwa in 5 Mikroliter Wasser 3 mg Peroxid löst.

Patentansprüche

1. Proberöhrchen für die Untersuchung von Proben im klinischen Bereich, insbesondere von Urinproben, unter Verwendung eines ersten Reagenz, das mit einer in der Probe vorhandenen festzustellenden Substanz unter Abgabe von $H_2O_2$ reagiert, und eines zweiten Reagenz, das mit Hilfe des derart gebildeten $H_2O_2$ die Oxidation eines Farbstoffbildners zum Farbstoff bewirkt, der ein Indikator für die festzustellende Substanz ist, dadurch gekennzeichnet, daß zur Ausschaltung von Störungen, die von weiteren in der Probe enthaltenen mit $H_2O_2$ reagierenden Substanzen verursacht werden, das Proberöhrchen
   a) Katalase,
   b) eine unter dem Einfluß von Katalase Sauerstoff abgebende Substanz, sowie
   c) eine Oxidase, die in Gegenwart des Sauerstoffs die Störungssubstanzen oxidiert,

   enthält, wobei die Katalase und die Oxidase eine Schicht (2) bilden bzw. in einer Schicht eingebettet sind, die einen Teil (h) der Innenwandung (2) des Röhrchens (1) bedeckt, und daß ferner die Sauerstoff abgebende Substanz (5) als Trockensubstanz in dem Röhrchen enthalten ist.

2. Proberöhrchen nach Anspruch 1 für die Untersuchung von Urinproben mit Hilfe von Glucose-Teststreifen, die als erstes Reagenz Glucose-Oxidase und als zweites Reagenz Peroxidase enthalten, dadurch gekennzeichnet, daß zur Ausschaltung von Störungen durch in der Urinprobe enthaltene Ascorbinsäure, das Röhrchen als eine unter dem Einfluß von Katalase Sauerstoff abgebende Substanz Peroxid und als Oxidase Ascorbinsäure-Oxidase enthält.

3. Proberöhrchen nach Anspruch 1 oder 2, dadurch gekennzeichnet, daß das Peroxid in dem Röhrchen (1) als trockenes Pulver enthalten ist.

4. Proberöhrchen nach Anspruch 1 oder 2, dadurch gekennzeichnet, daß das Peroxid im Röhrchen (1) als Beschichtung eines Peroxid-Trägers (6) enthalten ist.

5. Proberöhrchen nach Anspruch 1 oder 2, dadurch gekennzeichnet, daß das Peroxid an der im Inneren des Glasröhrchens (1) zugewandten Stirnfläche (4) eines das Glasröhrchen (1) verschließenden Stopfens (3) in Form einer Anhäufung (5) angetrocknet ist.

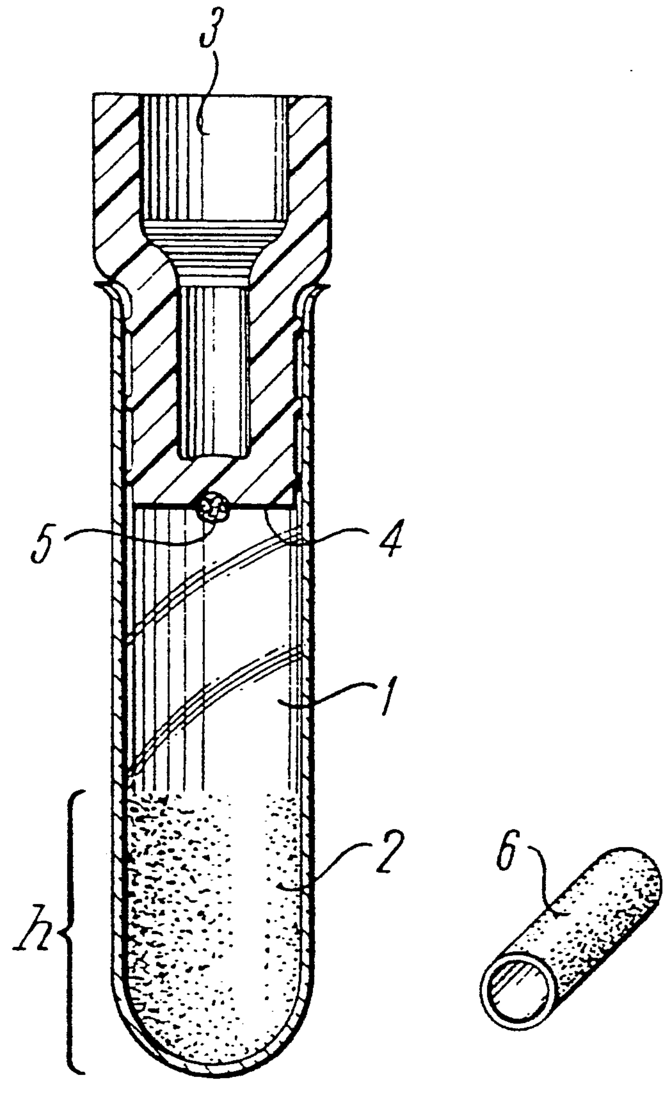
3
5
4
1
2
6
h

0014898

Europäisches Patentamt

**EUROPÄISCHER RECHERCHENBERICHT**

Nummer der Anmeldung
EP 80 10 0597

| EINSCHLÄGIGE DOKUMENTE | | | KLASSIFIKATION DER ANMELDUNG (Int.Cl. 3) |
|---|---|---|---|
| Kategorie | Kennzeichnung des Dokuments mit Angabe, soweit erforderlich, der maßgeblichen Teile | betrifft Anspruch | |
| X | DE - A - 2 630 043 (INSTITUT FRESENIUS CHEMISCHE UND BIOLOGISCHE LABORATORIEN GmbH)<br>* Seite 5, Zeilen 4-17; Seite 6, Zeilen 1-25 *<br>-- | 1,2 | C 12 Q 1/30<br>1/26<br>1/54 |
| | US - H - T 978 003 (TAI WING WU)<br>* Zusammenfassung *<br>-- | 1 | |
| | FR - A - 2 354 561 (BOEHRINGER MANNHEIM GmbH)<br>* Seite 2, Zeilen 7-40; Seite 3, Zeilen 1-40; Seite 4, Zeilen 1-10; Beispiel 8; Ansprüche 1-3, 5,8,9,15 *<br>-- | 2 | RECHERCHIERTE SACHGEBIETE (Int. Cl. 3)<br>C 12 Q 1/00<br>1/26<br>1/28<br>1/30<br>1/54<br>G 01 N 33/66 |
| | FR - A - 2 363 107 (EASTMAN KODAK COMP.)<br>* Seite 2, Zeilen 30-42; Seite 3, Zeilen 1-40; Seite 4, Zeilen 1-31; Seite 5, Zeilen 1-14; Seite 9, Zeilen 16-39; Beispiel 1; Ansprüche 1,3,4,6,8,11,17,19 *<br>-- | 1,2 | |
| A | RESEARCH DISCLOSURE, Nr. 160, August 1977, Ref. Nr. 16032, "Reduction of ascorbic acid interference in assays of aqueous fluids", Seiten 24-28<br>* Der vollständige Artikel *<br>-- | 2 | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument |
| A | BE - A - 665 452 (MILES LAB.)<br>./. | 1 | |
| X | Der vorliegende Recherchenbericht wurde für alle Patentansprüche erstellt. | | &: Mitglied der gleichen Patentfamilie, übereinstimmendes Dokument |

| Recherchenort | Abschlußdatum der Recherche | Prüfer |
|---|---|---|
| Den Haag | 25-04-1980 | DE LUCA |

EPA form 1503.1 06.78

Europäisches Patentamt

# EUROPÄISCHER RECHERCHENBERICHT

Nummer der Anmeldung
EP 80 10 0597
-2-

| EINSCHLÄGIGE DOKUMENTE | | | KLASSIFIKATION DER ANMELDUNG (Int.Cl. 3) |
|---|---|---|---|
| Kategorie | Kennzeichnung des Dokuments mit Angabe, soweit erforderlich, der maßgeblichen Teile | betrifft Anspruch | |
| | * Seite 2, Zeilen 15-31; Seite 3, Zeilen 1-31; Seite 4, Zeilen 1-31; Seite 5, Zeilen 1-31; Seite 6, Zeilen 1-6 *<br>-- | | |
| A | METHODEN DER ENZYMATISCHEN ANALYSE Band II, 1974, Weinheim, DE, H.U. BERGMEYER et al.: "Bestimmung mit Glucose-Oxydase und Peroxydase" Seiten 1250-1259<br>* Seite 1253 *<br>-- | 1,2 | RECHERCHIERTE SACHGEBIETE (Int. Cl. 3) |
| A | METHODEN DER ENZYMATISCHEN ANALYSE Band 1, 1974, Weinheim, DE, HUGO AEBI: "Katalase", Seiten 713-716<br>* Seite 713 *<br>---- | 1 | |